# EUROPÄISCHE PATENTSCHRIFT

(11) **EP 1 432 827 B1**
(45) Veröffentlichungstag und Bekanntmachung des Hinweises auf die Patenterteilung: **19.11.2008**
(21) Anmeldenummer: 02776808.4
(22) Anmeldetag: 04.10.2002
(51) Int. Cl.: C12Q 1/68

(54) **VERFAHREN ZUM NACHWEIS VON DNA-METHYLIERUNG MITTELS MARKIERTEN S-ADENOSYLMETHIONINANALOGA**
METHOD FOR DETECTING DNA METHYLATION USING LABELLED S-ADENOSYLMETHIONINE ANALOGS
PROCEDE POUR DETECTER LA METHYLATION DE L'ADN AU MOYEN D'ANALOGUES MARQUES DE LA S-ADENOSYLMETHIONINE

(30) Priorität: 05.10.2001 DE 10151069
(43) Veröffentlichungstag der Anmeldung: 30.06.2004
(73) Patentinhaber: Epigenomics AG, 10178 Berlin (DE); Qiagen GmbH, 40724 Hilden (DE)
(72) Erfinder: BERLIN, Kurt, 14532 Stahnsdorf (DE); RIBBE, Joachim, Dr., 40231 Düsseldorf (DE)
(74) Vertreter: Schubert, Klemens
(86) Internationale Anmeldenummer: PCT/DE2002/003844
(87) Internationale Veröffentlichungsnummer: WO 2003/031648

(56) Entgegenhaltungen:
- WO-A-92/06985
- WO-A-99/10540
- WO-A-02/101353
- WO-A2-01/27317
- DE-A1- 19 935 772
- DATABASE BIOSIS [Online] BIOSCIENCES INFORMATION SERVICE, PHILADELPHIA, PA, US; Oktober 1998 (1998-10) FOWLER BRENDA M ET AL: "Hypomethylation in cervical tissue: Is there a correlation with folate status?" Database accession no. PREV199800498186 XP002251320 & CANCER EPIDEMIOLOGY BIOMARKERS & PREVENTION, Bd. 7, Nr. 10, Oktober 1998 (1998-10), Seiten 901-906, ISSN: 1055-9965
- "Fluorescent DNA labeling by PCR" AMERSHAM BIOSCIENCES APPLICATION NOTE, Nr. 62, 1999, Seiten 1-8,
- BING ZHU ET AL: "Overexpression of 5-methylcytosine DNA glycosylase in human embryonic kidney cells EcR293 demethylates the promoter of a hormone-regulated reporter gene" PNAS, 24. März 2001 (2001-03-24), Seiten 5031-5036,
- JOST JEAN-PIERRE ET AL: "Mechanisms of DNA Demethylation in Chicken Embryos: Purification and Properties of a 5-Methylcytosine-DNA glycosylase" JOURNAL OF BIOLOGICAL CHEMISTRY, Bd. 270, Nr. 17, 1995, Seiten 9734-9739,
- FREMONT M. ET AL: 'Demethylation of DNA by purified chick embryo 5-methylcytosine-DNA glycosylase requires both protein and RNA' NUCLEIC ACIDS RESEARCH Bd. 25, Nr. 12, 1997, Seiten 2375 - 2380
- BHATTACHARYA S.K. ET AL: 'A mammalian protein with specific demethylase activity for mCpG DNA' NATURE Bd. 397, Nr. 6720, 1999, LONDON, Seiten 579 - 583
- BING ZHU ET AL: "5-Methylcytosine-DNA glycosylase activity is present in a cloned G/T mismatch DNA glycosylase associated with the chicken embryo DNA demethylation complex" PNAS, Bd. 97, Nr. 10, 2000, Seiten 5135-5139,
- VAIRAPANDI M. ET AL: 'Human DNA-demethylating activity: A glycosylase associated with RNA and PCNA' JOURNAL OF CELLULAR BIOCHEMISTRY Bd. 79, Nr. 2, 2000, Seiten 249 - 260
- BING ZHU ET AL: "5-Methylcytosine DNA glycosylase activity is also present in the human MBD4 (G/T mismatch glycosylase) and in a related avian sequence" NUCLEIC ACIDS RESEARCH, Bd. 28, Nr. 21, 2000,

## Beschreibung

Die vorliegende Erfindung betrifft ein Verfahren zum Nachweis von DNA-Methylierung, insbesondere an Cytosin und Adenin, in DNA-Proben.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern äußern sich pathogene Zustände in einem veränderten Methylierungsmuster einzelner Gene oder des Genoms.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neue und die mittlerweile am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällungs- und Reinigungsschritte durch schnelle Dialyse ersetzt (Olek A, Oswald J, Walter J. A modified and improved method for bisulphate based cytosine methylation analysis. Nucleic Acids Res. 1996 DEC 15;24(24):5064-6). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden: Rein T, DePamphilis ML, Zorbas H. Identifying 5-methylcytosine and related modifications in DNA genomes. Nucleic Acids Res. 1998 May 15;26 (10) : 2255-64.

Die Bisulfit-Technik wird bisher bis auf wenige Ausnahmen (z. B. Zeschnigk M, Lich C, Buiting K, Dörfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) nur in der Forschung angewendet. Immer aber werden kurze, spezifische Stücke eines bekannten Gens nach einer Bisulfit-Behandlung amplifiziert und entweder komplett sequenziert (Olek A, Walter J. The pre-implantation ontogeny of the H19 methylation imprint. Nat Genet. 1997 Nov. ; 17 (3) : 275-6) oder einzelne Cytosin-Positionen durch eine "Primer-Extension-Reaktion" (Gonzalgo ML, Jones PA. Rapid quantitation of methylation differences at specific sites using methylation-sensitive single nucleotide primer extension (Ms-SNuPE). Nucleic Acids Res. 1997 Jun. 15 ; 25 (12) : 2529-31, WO-Patent 9500669) oder einen Enzymschnitt (Xiong Z, Laird PW. COBRA: a sensitive and quantitative DNA methylation assay. Nucleic Acids Res. 1997 Jun. 15 ; 25 (12) : 2532-4) nachgewiesen. Zudem ist auch der Nachweis durch Hybridisierung beschrieben worden (Olek et al., WO 99/28498).

Harnstoff verbessert die Effizienz der Bisulfit-Behandlung vor der Sequenzierung von 5-Methylcytosin in genomischer DNA (Paulin R, Grigg GW, Davey MW, Piper AA. Urea improves efficiency of bisulphate-mediated sequencing of 5'-methylcytosine in genomic DNA. Nucleic Acids Res. 1998 Nov. 1; 26(21): 5009-10).

Weitere Publikationen, die sich mit der Anwendung der Bisulfit-Technik zum Methylierungsnachweis bei einzelnen Genen befassen, sind:
Grigg G, Clark S. sequencing 5-methylcytosine residues in genomic DNA. Bioassays. 1994 Jun.; 16(6): 431-6, 431; Zeschnigk M, Schmitz B, Dittrich B, Buiting K, Horsthemke B, Dörfler W. Imprinted segments in the human genome: different DNA methylation patterns in the Prader-Willi/Angelman syndrome region as determined by the genomic sequencing method. Hum Mol Genet. 1997 Mar; 6(3): 387-95; Feil R, Charlton J, Bird AP, Walter J, Reik W. Methylation analysis on individual chromosomes: improved protocol fort bisulphate genomic sequencing. Nucleic Acids Res. 1994 Feb. 25;22(4):695-6; Martin V, Ribieras S, Song-Wang X, Rio MC, Dante R. Genomic sequencing indicates a correlation between DNA hypomethylation in the 5' region of the pS2 gene andin its expression in human breast cancer cell lines. Gene. 1995 May 19;157(1-2):261-4; WO 97 46705, WO 95 15373 und WO 45560.

Ein weiteres bekanntes Verfahren ist die sogenannte methylierungssensitive PCR (Herman JG, Graff JR, Myohanen S, Nelkin BD, Baylin SB. (1996), Methylation-specific PCR: a novel PCR assay for methylation status of CpG islands. Proc Natl Acad Sci USA. Sep 3;93(18):9821-6). Für dieses Verfahren werden Primer verwendet, die entweder nur an eine Sequenz hybridisieren, die durch die Bisulfit-Behandlung einer an der betreffenden Position unmethylierten DNA entsteht, oder aber umgekehrt Primer, welche nur an eine Nukleinsäure bindet, die durch die Bisulfit-Behandlung einer an der betreffenden Position unmethylierten DNA entsteht. Mit diesen Primern können demnach Amplifikate erzeugt werden, deren Detektion wiederum Hinweise auf das Vorliegen einer methylierten oder unmethylierten Position in der Probe liefern, an welche die Primer binden.

Ein neueres Verfahren ist auch der Nachweis von Cytosin-Methylierung mittels einer Taqman PCR, das als Methyl-Light bekannt geworden ist (WO 00/70090). Mit diesem Verfahren ist es möglich, den Methylierungsstatus einzelner oder weniger Positionen direkt im Verlauf der PCR nachzuweisen, so dass sich eine nachfolgende Analyse der Produkte erübrigt.

Eine Übersicht über den Stand der Technik in der Oligomer Array Herstellung lässt sich aus einer im Januar 1999 erschienenen Sonderausgabe von Nature Genetics (Nature Genetics Supplement, Volume 21, January 1999), der dort zitierten Literatur und dem US-Patent 5994065 über Methoden zur Herstellung von festen Trägern für Zielmoleküle wie Oligonucleotide bei vermindertem nichtspezifischen Hintergrundsignal entnehmen.

Für die Abtastung eines immobilisierten DNA-Arrays sind vielfach fluoresziert markierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Matrix-assistierte Laser Desorptions/Ionisations-Massenspektro-metrie (MALDI-TOF) ist eine sehr leistungsfähige Entwicklung für die Analyse von Biomolekülen (Karas M, Hillenkamp F. Laser desorption ionization of proteins with molecular masses exceeding 10,000 daltons. Anal Chem. 1988 Oct. 15;60(20):2299-301). Ein Analyt wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

MALDI-TOF Spektroskopie eignet sich ausgezeichnet zur Analyse von Peptiden und Proteinen. Die Analyse von Nukleinsäuren ist etwas schwieriger (Gut, I. G. und Beck, S. (1995), DNA and Matrix Assisted Laser Desorption Ionization Mass Spectrometry. Molecular Biology: Current Innovations and Future Trends 1: 147-157.) Für Nukleinsäuren ist die Empfindlichkeit etwa 100 mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter. In der MALDI-TOF Spektroskopie spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlerweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I. G. und Beck, S. (1995), A procedure for selective DNA alkylation and detection by mass spectrometry. Nucleic Acids Res. 23: 1367-1373). Die Kopplung eines "charge tags" an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Ständardmethodik findet sich in Referenzen wie Fritsch und Maniatis, Molecular Cloning: A Laboratory Manual, 1989.

Nach der Erfindung der PCR sind in den folgenden Jahren zahlreiche Varianten bekannt geworden, die diese Technik zur Amplifikation der DNA verfeinern. Insbesondere ist hier die Multiplexierung der PCR (Multiplex-PCR) zu erwähnen, wobei man mehr als 2 spezifische Primer einsetzt und dabei in einem Reaktionsgefäß eine Vielzahl von verschiedenen, spezifischen Amplifikationen erzeugen kann. Besonders interessant ist auch die sogenannte Nested PCR, welche unter anderem zum Nachweis besonders geringer DNA Mengen verwendet wird. Diese Art der PCR besteht aus zwei aufeinanderfolgenden Amplifikationen, wobei die Primer der zweiten Amplifikation innerhalb des ersten Amplifikates liegen und nicht mit den Primern der ersten Amplifikation identisch sind. Dadurch wird eine besondere Spezifität erreicht, da die Primer der zweiten Amplifikation nur dann funktionieren, wenn in der ersten Amplifikation das beabsichtigte Fragment erzeugt wurde. Dagegen ist die die Vermehrung etwaiger Nebenprodukte der ersten Amplifikation in der zweiten so gut wie ausgeschlossen.

Die WO 92/06985 A beschreibt ein Verfahren zur Detektion des Methylierungsstatus unter Verwendung von DNA-Methyltransferase und (3H)-5-Adenosylmethionin.

Brenda M. Fowler et al. ("Hypomethylation in cervical tissue: Is there a correlation with folate status?", Database Bios [Online] Biosciences Information Service, Philadelphia, PA, US; Oktober 1998 (1988-10)) beschreiben ein Verfahren zur Detektion der Hypomethylierung eines. Gewebes durch Verwendung einer DNA-Methyltransferase und eines (3-H)Methyl-S-adenosylmethionin.

Ferner wird in der WO 02/101353 A ein Verfahren und Produkte zur Analyse von Nukleinsäuren auf Grundlage des Methylierungsstatus beschrieben.

Die WO 01/27317 A2 (Epigenomics AG) beschreibt ein Verfahren zur Untersuchung von 5-Position Methylierungsänderungen.

Des Weiteren wird in der DE 199 35 772 A1 (Epigenomics GmbH) ein Verfahren zur relativen Quantifizierung der Methylierung von Cytosin Basen in amplifizierten Nukleinsäurefragmenten beschrieben.

Es sind demnach bislang vielerlei Verfahren zur Methylierungsanalyse Stand der Technik. Gegenstand der vorliegenden Erfindung ist ein Verfahren zur Detektion von Cytosin- Methylierungen gemäß dem kenzeichnemden Teil des Anspruchs 1. Die vorliegende Erfindung soll jedoch eine Möglichkeit zur Analyse des Methylierungsgrades in einem genomischen DNA-Abschnitt bereitstellen. Dabei ist es bevorzugt nicht erforderlich, eine Polymerasereaktion durchzuführen, was die Durchführung des Verfahrens erleichtert. Es ist im Rahmen einer Methylierungsanalyse im Bereich der klinischen Diagnostik wesentlich, dass Untersuchungsergebnisse möglichst schnell zur Verfügung gestellt werden können und dass sich der experimentelle Aufwand in möglichst engen Grenzen hält. Dazu ist das hier beschriebene Verfahren in besonderem Masse geeignet.

Das hier beschriebene Verfahren zur Detektion von Cytosin-Methylierung besteht in der Kombination der folgenden Schritte:

Zuerst wird die genomische DNA Probe wahlweise mit einem Restriktionsenzym behandelt, so dass diese aus kleineren Fragmenten, bevorzugt in der Grössenordnung zwischen 2 kb und 80 kb besteht. Die DNA Probe wird daraufhin mit einem Bisulfit und einem Radikalfänger, der die Zersetzung der DNA verhindern soll, behandelt und die enstandenen Bisulfitaddukte alkalisch hydrolysiert. Dabei werden alle methylierten Cytosine im wesentlichen unverändert bleiben, während die nicht methylierten Cytosinbasen in Uracil umgewandelt werden.
Nun kann durch Verwen dung einer Methyltransferase im nächsten Schritt festgestellt werden, inwieweit diese Umsetzung an gegebenen Positionen stattgefunden hat. So ist es möglich, mit einer Methyltransferase Sss1, welche selektiv die Sequenz 5'CpG-3' methyliert, unter Verwendung eines modifizierten S-Adenosylmethioninderivats zu unterscheiden, ob sich nun an dieser Position nach wie vor ein Cytosin befindet oder ob es in Uracil umgewandelt wurde, da nur die aufmethylierten Positionen durch das modifizierte S-Adenosylmethioninderivat und beispielsweise Sss1 fluoreszenzmarkiert werden können. Andere Enzyme sind sequenzsezifischer als Sss1 und daher kann man auch zusätzliche Schlüsse auf den Sequenzkontext ziehen. Da diese Positionen jedoch auch nach der Bisulfitbehandlung noch methyliert vorliegen, muss zuvor eine Demethylierung erfolgen. Dies wird bevorzugt mittels einer Kopierreaktion oder aber einer PCR erreicht. Dies hat zudem den Vorteil, dass man genau weiß, welche Abschnitte der genomischen DNA Probe im folgenden auf Methylierung untersucht werden.

Das oben erwähnte modifizierte S-Adenosylmethioninderivat hat die Eigenschaft, das sonst als Methylgruppendonor fungierende S-Adenosylmethionin in der enzymatischen DNA-Methylierung derart zu ersetzen, dass die Methylgruppe durch ein entsprechendes fluoreszenzmarkiertes Analogon ersetzt ist. Die Methyltransferase überträgt also anstelle einer Methylgruppe ein entsprechend fluoreszenzmarkiertes Analogon.

Die erfindungsgemäß verwendbaren modifizierten S-Adenosylmethioninderivate sind in ans ich bekannter Weise herstellbar. Dabei werden derartige Derivate hergestellt, bei denen der Fluorophor an sich bekannt ist. Geeignete Fluoreszenz gebende Moleküle sind dem Fachmann geläufig. Diese Fluoreszenz gebenden Moleküle werden dann in geeigneter Weise mit S-Adenosylmethionin verknüpft. Diese geschieht insbeondere in das Art, dass dabei Verbindungen entstehen, welche bei der Übertragung der Methylgruppen gleichzeitig die Fluoreszenz gebende Gruppe mit übertragen.

Nach einer PCR werden also die Amplifikate mit dem modifizierten S-Adenosylmethioninderivat und einer Methyltransferase sequenz-spezifisch fluoreszenzmarkiert, und zwar nur dann, wenn an den betreffenden Positionen zuvor eine Methylierung in der genomischen DNA Probe vorlag. Wird nun mit herkömmlichen Methoden wie (Kapillar) Gelelektrophorese, Chromatographie oder ähnlichen Verfahren eine Fragmentanalyse durchgeführt, so werden nur diejenigen Fragmente eine Fluoreszenzmarkierung tragen, die zuvor an der untersuchten Position methyliert vorlagen.

In einer besonders bevorzugten Variante des Verfahrens werden auf diese Art mehrere Fragmente gleichzeitig und besonders bevorzugt auch mehrere Positionen in diesen Fragmenten gleichzeitig auf Methylierung hin untersucht. Dies kann durch nacheinanderfolgende Markierung mit unterschiedlichen Farbstoffen an dem modifizierten S-Adenosylmethioninderivat in Verbindung mit unterschiedlichen sequenzspezifischen Methyltransferasen erfolgen.

Auch ist es möglich, in der PCR mit mehreren Primerpaaren gleichzeitig zu arbeiten und so eine Multiplex PCR auszuführen. Haben die dabei erzeugten Amplifikate verschiedene Längen, so ist eine gleichzeitige Analyse mittels einer der oben genannten Methoden möglich.

Eine weitere Möglichkeit der DNA-Methylierungsanalyse besteht in der Verwendung des modifizierten S-Adenosylmethioninderivats zusammen mit einer Methyltransferase wie Dnmt 1, welche für hemimethylierte Doppelstränge spezifisch ist und zu den sogenannten Maintenance-Methyltransferasen gehört. Bei diesem Verfahren macht man es sich zunutze, dass nach einer Kopierreaktion eines bestimmten Abschnittes der genomischen DNA-Probe selbiger Abschnitt an den Positionen hemimethyliert vorliegt, die in der genomischen DNA methyliert vorlagen, während die Positionen weiterhin unmethyliert vorliegen, welche vor der Kopierreaktion auch schon unmethyliert vorlagen. Das modifizierte S-Adenosylmethioninderivat in Verbindung mit Dnmt1 wird demnach nur an den zuvor methyliert vorliegenden Positionen selektiv eine Fluoreszenzmarkierung einbauen.

Diese kann nun mittels verschiedener Methoden sichtbar gemacht werden. Limitierend für diese Variante ist die fehlende exponentielle Amplifikation, welche die Verwendung von Dnmt1 in diesem Sinne unmöglich machen würde.

Dnmt1 kann jedoch auch in Verbindung mit dem modifizierten S-Adenosylmethioninderivat zum Nachweis unmethylierter Positionen in einem Pool von DNA Proben generell eingesetzt werden. Aus den doppelsträngigen DNA-Probenmolekülen werden dabei nach einem Schmelz- und Reannealingschritt Heteroduplexe gebildet. An den Positionen, an denen teilweise Methylierung vorlag, entstehen nach dem Reannealing hemimethylierte Doppelstränge, welche sich durch Dnmt 1 und modifizierte S-Adenosylmethioninderivate selektiv fluoreszenzmarkieren lassen. Dieses Verfahren kann zum einen zum Auffinden differentiell methylierter CpG Positionen verwendet werden. Zum anderen kann jedoch durch Beimischung unmethylierter DNA bevorzugt eine Fluoreszenzmarkierung dann erfolgen, wenn in einer Probe methylierte Positionen vorlagen. Zum anderen ist selektiv eine Fluoreszenzmarkierung von unmethylierter DNA möglich, wenn zuvor eine Beimischung einer aufmethylierten DNA Probe erfolgt.

Die für die vorstehenden Experimente erforderlichen methylierten und unmethylierten Standard-DNA Proben werden bevorzugt durch Aufmethylierung einer genomischen DNA-Probe mit Sss1 erzeugt. Für unmethylierte DNA wird bevorzugt DNA, welche aus peripherem Blut isoliert wurde, verwendet, die an den meisten zu untersuchenden Positionen unmethyliert vorliegt. Alternativ kann beispielsweise aus Spermien isolierte DNA verwendet werden.

Die fluoreszenzmarkierte genomische DNA kann wie oben beschrieben analysiert werden. Eine zusätzliche interessante Möglichkeit besteht darin, eine 2D Gelelektrophorese durchzuführen, bei welcher nun bei jedem Fragment zusätzlich Information über dessen Methylierungsstatus erhalten werden kann.

Eine weitere Verfahrensvariante besteht darin, dass modifizierte S-Adenosylmethioninderivate zur Übertragung von idealerweise fluoreszierenden Gruppen verwendet werden, welche eine nachfolgende Polymerasereaktion an dem so modifizierten DNA-Templat inhibieren. An den Positionen, an denen eine solche Gruppe eingebaut wurde, wird die Polymerasereaktion bevorzugt abbrechen. Dies ließe sich für die Durchführung einer Sequenzierreaktion, vergleichbar einer Sanger-Sequenzierung, nutzen, wobei in dieser Variante jedoch nicht bestimmte Basenabfolgen, sondern untern den jeweils gewählten Bedingungen methylierbare Positionen aufgezeigt würden. Die jeweils methylierbaren Positionen bestimmen sich zum einen nach dem schon vorhandenen Methylierungsstatus des Templats und zum anderen nach der Sequenzspezifität der verwendeten Methyltransferase.

Das folgende Beispiel erläutert die Erfindung.

### Beispiel:

Eine genomische DNA-Probe wurde mit dem Restriktionenzym Mss1 verdaut.
Für die Bisulfitreaktion wurde eine Natriumbisulfit-Lösung verwendet.
Die Desulfonierung erfolgte mit 50µl einer 50mM Tris-HCl-Lösung bei pH9 für 20 Minuten bei 96°C.
Die mit Bisulfit behandelte DNA wurde mit den Primeroligonukleotiden TAGGAAAAGGAGTTGGATTTTT (Seq-ID 1) und CCCCCTACCTAACCTATAATCA (Seq-ID 2) des Gens DAXX amplifiziert.

Die Amplifikation wurde folgendermaßen durchgeführt: 95°C - 15 Min, 40 Zyklen: 94°C - 1:00 Min, 55°C - 0:45 Min, 72°C - 1:30 Min und eine abschließende Verlängerung bei 72°C - 10 Min.

In einer anschließenden Reaktion wurde fluoreszenzmarkiertes S-Adenosylmethioninderivat, welches mit analogen Verfahren in an sich bekannter Weise hergestellt wurde, als Methylgruppendonator für die Methylase Sss I (NEB) verwendet. Dafür wurden 500 ng DNA unter Verwendung von 0,5 units Sss I sowie dem S-Adenosylmethioninderivat und dem zugehörigen Reaktionspuffer laut Herstellerangaben für 8 Stunden bei 37°C inkubiert.
Die durch die fluoreszenzmarkierten S-Adenosylmethioninderivate markierten Cytosine wurden in einer Fragmentanalyse nachgewiesen.

## Patentansprüche

1. Verfahren zur Detektion von Cytosin-Methylierungen, **dadurch gekennzeichnet, dass** folgende Schritte durchgeführt werden:
a) die zu untersuchende DNA wird mit Bisulfit behandelt, so dass methylierte Cytosine unverändert bleiben, während die nicht-methylierten Cytosine in Uracil umgewandelt werden,
b) die DNA wird demethyliert,
c) die DNA wird unter Verwendung einer Methyltransferase und eines modifizierten S-Adenosylmethioninderivats umgesetzt, so dass diejenigen Positionen markiert werden, an denen in der genomischen DNA eine Methylierung vorlag,
d) es wird festgestellt, inwieweit an gegebenen Positionen eine Umsetzung stattgefunden hat.

2. Verfahren nach Anspruch 1, **dadurch gekennzeichnet, dass** die DNA zunächst mit einem Restriktionsenzym behandelt wird.

3. Verfahren nach einem der oben genannten Ansprüchen, **dadurch gekennzeichnet, dass** als Methyltransferase Sss1 verwendet wird.

4. Verfahren nach einem der oben genannten Ansprüche, **dadurch gekennzeichnet, dass** die Demethylierung mittels einer Kopierreaktion oder einer PCR erfolgt.

5. Verfahren nach einem der oben genannten Ansprüche, **dadurch gekennzeichnet, dass** ein fluoreszenzmarkiertes S-Adenosylmethioninderivat eingesetzt wird.

6. Verfahren nach einem der oben genannten Ansprüche, **dadurch gekennzeichnet, dass** Schritt d) mittels (Kapillar)-Gelelektrophorese, Chromatographie oder einem ähnlichen Verfahren durchgeführt wird.

7. Verfahren nach einem der oben genannten Ansprüche, **dadurch gekennzeichnet, dass** mehrere Fragmente gleichzeitig untersucht werden.

8. Verfahren nach einem der oben genannten Ansprüche, **dadurch gekennzeichnet, dass** mehrere Positionen gleichzeitig untersucht werden.

9. Verfahren nach einem der oben genannten Ansprüche, **dadurch gekennzeichnet, dass** die Markierung nacheinander mit unterschiedlichen - an die S-Adenosylmethioninderivate gebundenen- Farbstoffen in Verbindung mit unterschiedlichen sequenzspezifischen Methyltransferasen erfolgt.

10. Verfahren nach einem der oben genannten Ansprüche, **dadurch gekennzeichnet, dass** eine Multiplex PCR ausgeführt wird.

11. Verfahren zur Methylierungsanalyse, **dadurch gekennzeichnet, dass** folgende Schritte durchgeführt werden:
a) die genomische DNA wird durch eine Kopierreaktion so umgewandelt, dass die Positionen, die in der genomischen DNA methyliert waren, nun hemimethyliert vorliegen, während die Positionen weiterhin unmethyliert vorliegen, welche vor der Kopierreaktion schon unmethyliert vorlagen,
b) die DNA wird mit einem S-Adenosylmethioninderivate und einer Methyltransferase umgesetzt, die für hemimethylierte Doppelstränge spezifisch ist,
c) die modifizierte S-Adenosylmethioninderivate werden selektiv an den Positionen eingebaut, die zuvor methyliert vorlagen.

12. Verfahren nach Anspruch 11, **dadurch gekennzeichnet, dass** es sich bei der Methyltransferase um Dnmt 1 handelt.

13. Verfahren zur Analyse von Cytosinmethylierungen in einem Pool von DNA-Proben, **dadurch gekennzeichnet, dass** folgende Schritte durchgeführt werden:
a) ein Schmelz- und Re-Annealingsschritt unter Bildung von hemimethylierten Heteroduplexen,
b) eine Markierung der unmethylierten Positionen durch Dnmt1 und modifizierte S-Adenosylmethioninderivate.

14. Verfahren nach Anspruch 13, **dadurch gekennzeichnet, dass** eine Beimischung unmethylierter DNA erfolgt.

15. Verfahren nach Anspruch 13, **dadurch gekennzeichnet, dass** eine Beimischung aufmethylierter DNA erfolgt.

16. Verfahren nach einem der oben genannten Ansprüche, **dadurch gekennzeichnet, dass** eine Analyse über eine 2D- Gelelektrophorese erfolgt.

17. Verfahren nach einem der oben genannten Ansprüche, **dadurch gekennzeichnet, dass** modifizierte S-Adenosylmethioninderivate verwendet werden, welche eine nachfolgende Polymerasereaktion an dem DNA-Templat inhibieren.

18. Verfahren nach Anspruch 17, **dadurch gekennzeichnet, dass** anschließend eine Sequenzierreaktion durchgeführt wird.

## Claims

1. Method for detecting cytosine methylations, **characterized in that** the following steps are carried out:
a) the DNA to be investigated is treated with bisulphite so that methylated cytosines remain unchanged, while the unmethylated cytosines are converted into uracil,
b) the DNA is demethylated,
c) the DNA is converted with use of a methyltransferase and of a modified S-adenosylmethionine derivative so as to label those positions at which a methylation was present in the genomic DNA,
d) the extent to which a conversion has taken place at given positions is established.

2. Method according to Claim 1, **characterized in that** the DNA is initially treated with a restriction enzyme.

3. Method according to either of the abovementioned claims, **characterized in that** Sss1 is used as methyl transferase.

4. Method according to any of the abovementioned claims, **characterized in that** the demethylation is effected by means of a copying reaction or of a PCR.

5. Method according to any of the abovementioned claims, **characterized in that** a fluorescence-labelled S-adenosylmethionine derivative is employed.

6. Method according to any of the abovementioned claims, **characterized in that** step d) is carried out by means of (capillary) gel electrophoresis, chromatography or a similar method.

7. Method according to any of the abovementioned claims, **characterized in that** a plurality of fragments are investigated simultaneously.

8. Method according to any of the abovementioned claims, **characterized in that** a plurality of positions are investigated simultaneously.

9. Method according to any of the abovementioned claims, **characterized in that** the labelling takes place successively with different dyes - bound to the S-adenosylmethionine derivatives - in conjunction with different sequence-specific methyltransferases.

10. Method according to any of the abovementioned claims, **characterized in that** a multiplex PCR is carried out.

11. Method for methylation analysis, **characterized in that** the following steps are carried out:
a) the genomic DNA is transformed by a copying reaction so that the positions which were methylated in the genomic DNA are now hemimethylated, while the positions already unmethylated before the copying reaction remain unmethylated,
b) the DNA is converted with an S-adenosylmethionine derivative and a methyltransferase which is specific for hemimethylated double strands,
c) the modified S-adenosylmethionine derivatives are selectively incorporated at the positions which were previously methylated.

12. Method according to Claim 11, **characterized in that** the methyltransferase is Dnmt 1.

13. Method for analysing cytosine methylations in a pool of DNA samples, **characterized in that** the following steps are carried out:
a) a melting and reannealing step to form hemimethylated heteroduplexes,
b) a labelling of the unmethylated positions by Dnmt 1 and modified S-adenosylmethionine derivatives.

14. Method according to Claim 13, **characterized in that** unmethylated DNA is admixed.

15. Method according to Claim 13, **characterized in that** DNA which has undergone methylation is admixed.

16. Method according to any of the abovementioned claims, **characterized in that** an analysis takes place by 2D gel electrophoresis.

17. Method according to any of the abovementioned claims, **characterized in that** modified S-adenosylmethionine derivatives which inhibit a subsequent polymerase reaction on the DNA template are used.

18. Method according to Claim 17, **characterized in that** a sequencing reaction is subsequently carried out.

## Revendications

1. Procédé pour la détection de méthylations de la cytosine, **caractérisé en ce qu'**on met en oeuvre les étapes suivantes :
(a) on traite l'ADN à étudier avec du bisulfite, de telle sorte que les cytosines méthylées restent inchangées, tandis que les cytosines non méthylées sont converties en uracile,
(b) on déméthyle l'ADN,
(c) on fait réagir l'ADN, par utilisation d'une méthyltransférase et d'un dérivé de S-adénosylméthionine modifié, de façon à marquer les positions sur lesquelles on avait une méthylation dans l'ADN génomique,
(d) on note dans quelle mesure une réaction a eu lieu sur les positions indiquées.

2. Procédé selon la revendication 1, **caractérisé en ce que** l'ADN est d'abord traité avec une enzyme de restriction.

3. Procédé selon l'une des revendications précédentes, **caractérisé en ce qu'**on utilise en tant que méthyltransférase la Sss1.

4. Procédé selon l'une des revendications précédentes, **caractérisé en ce que** la déméthylation est réalisée à l'aide d'une réaction de copie ou d'une PCR.

5. Procédé selon l'une des revendications précédentes, **caractérisé en ce qu'**on utilise un dérivé de S-adénosylméthionine marqué par fluorescence.

6. Procédé selon l'une des revendications précédentes, **caractérisé en ce que** l'étape d) est mise en oeuvre par une électrophorèse (capillaire) sur gel, une chromatographie ou un procédé analogue.

7. Procédé selon l'une des revendications précédentes, **caractérisé en ce que** l'on étudie simultanément plusieurs fragments.

8. Procédé selon l'une des revendications précédentes, **caractérisé en ce qu'**on étudie simultanément plusieurs positions.

9. Procédé selon l'une des revendications précédentes, **caractérisé en ce que** le marquage s'effectue successivement avec des colorants différents - liés aux dérivés de S-adénosylméthionine - en liaison avec différentes méthyltransférases spécifiques de séquence.

10. Procédé selon l'une des revendications précédentes, **caractérisé en ce qu'**on procède à une PCR multiplex.

11. Procédé pour analyse de la méthylation, **caractérisé en ce qu'**on met en oeuvre les étapes suivantes :
a) on convertit l'ADN génomique par une réaction de copie de telle sorte que les positions qui ont été méthylées dans l'ADN génomique se présentent maintenant sous forme hémi-méthylée, tandis que les positions qui avant la réaction de copie étaient déjà non méthylées se présentent encore sous forme non méthylée,
b) on fait réagir l'ADN avec un dérivé de S-adénosylméthionine et une méthyltransférase qui est spécifique des doubles brins hémi-méthylés,
c) on incorpore sélectivement les dérivés de S-adénosylméthionine modifiés sur les positions qui auparavant étaient méthylées.

12. Procédé selon la revendication 11, **caractérisé en ce que** la méthyltransférase est la Dnmt 1.

13. Procédé pour l'analyse de méthylations de la cytosine dans un pool d'échantillons d'ADN, **caractérisé en ce qu'**on met en oeuvre les étapes suivantes :
a) une étape de fusion ou de ré-annelage, avec formation d'hétéroduplex hémi-méthylés,
b) un marquage des positions non méthylées par de la Dnmt1 et des dérivés de S-adénosylméthionine modifiés.

14. Procédé selon la revendication 13, **caractérisé en ce qu'**on procède à l'addition, par mélange, d'ADN non méthylé.

15. Procédé selon la revendication 13, **caractérisé en ce qu'**on procède à une addition, par mélange, d'un ADN qui a été méthylé.

16. Procédé selon l'une des revendications précédentes, **caractérisé en ce qu'**on procède à une analyse par électrophorèse sur gel 2D.

17. Procédé selon l'une des revendications précédentes, **caractérisé en ce qu'**on utilise des dérivés de S-adénosylméthionine modifiés qui inhibent une réaction ultérieure par polymérase sur la matrice d'ADN.

18. Procédé selon la revendication 17, **caractérisé en ce qu'**on met en oeuvre ensuite une réaction de séquençage.
